(19) Europäisches Patentamt
European Patent Office
Office européen des brevets

(11) EP 0 915 836 B1

(12) **FASCICULE DE BREVET EUROPEEN**

(45) Date de publication et mention de la délivrance du brevet:
**05.06.2002 Bulletin 2002/23**

(21) Numéro de dépôt: **97935616.9**

(22) Date de dépôt: **23.07.1997**

(51) Int Cl.[7]: **C07C 251/22**, H01F 1/42, C07D 209/48

(86) Numéro de dépôt international:
**PCT/FR97/01381**

(87) Numéro de publication internationale:
**WO 98/04519 (05.02.1998 Gazette 1998/05)**

(54) **MATERIAUX A PROPRIETES FERROMAGNETIQUES COMPORTANT DES MOLECULES ORGANIQUES A PROPRIETES MAGNETIQUES ET LEURS PROCEDES DE FABRICATION**

MATERIALEN MIT FERROMAGNETISCHEN EIGENSCHAFTEN, ORGANISCHEN VERBINDUNGEN MIT FERROMAGNETISCHEN EIGENSCHAFTEN ENTHALTEND UND VERFAHREN ZU DEREN HERSTELLUNG

MATERIALS WITH FERROMAGNETIC PROPERTIES COMPRISING ORGANIC MOLECULES WITH MAGNETIC PROPERTIES, AND METHODS FOR MAKING SAME

(84) Etats contractants désignés:
**BE CH DE DK ES FR GB IT LI NL**

(30) Priorité: **26.07.1996 FR 9609476**

(43) Date de publication de la demande:
**19.05.1999 Bulletin 1999/20**

(73) Titulaire: **GEMPLUS**
**13881 Gémenos Cédex (FR)**

(72) Inventeurs:
- **LERICHE, Christian**
  **F-13530 Trets (FR)**
- **LE MEHAUTE, Alain**
  **F-91190 Gif-sur-Yvette (FR)**

(56) Documents cités:

| | |
|---|---|
| **EP-A- 0 512 926** | **EP-A- 0 645 414** |
| **EP-A- 0 680 989** | **DE-A- 1 620 055** |
| **DE-B- 1 254 120** | **FR-A- 2 121 101** |
| **FR-A- 2 234 277** | **FR-A- 2 262 022** |
| **FR-A- 2 262 024** | **FR-A- 2 684 677** |
| **US-A- 3 253 022** | |

- **SMITH W F, JR. ET AL: "Quenching of singlet molecular oxygen in solution by azomethine dyes" J. AM. CHEM. SOC. (JACSAT);75; VOL.97 (10); PP.2764-70, EASTMAN KODAK CO.;RES. LAB.; ROCHESTER; N. Y., XP002030135**
- **SERVE D: "Les propriétés électrochimiques des diphénylamines et de leur produits d'oxydation en milieu organique. V. Cyanation anodique des diphénylamines polysubstitutées" BULL. SOC. CHIM. FR. (BSCFAS);76; (11-12, PT. 2); PP.1993-8, CEN;DEP. RECH. FONDAM.; GRENOBLE; FR., XP002030136**
- **HERKSTROETER W G: "Determination of triplet-energy levels in azomethine dyes by energy-transfer measurements" J. AM. CHEM. SOC. (JACSAT);75; VOL.97 (11); PP.3090-6, EASTMAN KODAK CO.;RES. LAB.; ROCHESTER; N. Y., XP002030137**
- **BAKER P B ET AL: "Studies in peroxidase action. XXIII. Oxidation of polymethylated anilines" TETRAHEDRON (TETRAB);74; VOL.30 (2); PP.337-41, UNIV. CHEM. LAB.;CAMBRIDGE; ENGL., XP002030138**
- **GRIFFITHS J ET AL: "Cyclopenta[b][1,4]benzoxazines. 1,2,3-Trichloro derivatives from hexachlorocyclopentadiene" CHEM. IND. (LONDON) (CHINAG);74; (9); PP.379-80, UNIV. LEEDS;DEP. COLOUR CHEM.; LEEDS; ENGL., XP000671442**
- **DATABASE XFIRE Beilstein Beilstein Registry Number 3473364, XP002030142 & LEIBERMANN; SCHULZE: JUSTUS LIEBIGS ANN. CHEM., vol. 5608, 1934, pages 144-151,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 915 836 B1

- **ATKINSON R S ET AL: "Thermal decomposition of 9-(substituted phenylimino)-1,4-dihydro-1, 2,3,4-tetraphenyl-1,4-methanonaphthalenes" J. CHEM. SOC., PERKIN TRANS. 1 (JCPRB4);74; (23); PP.2619-22, UNIV. LEICESTER;DEP. CHEM.; LEICESTER; ENGL., XP002030139**
- **SUZUKI Y ET AL: "Cycloaddition reactions of isocyanides with diphenylacetylene using transition-metal complexes" J. CHEM. SOC., CHEM. COMMUN. (JCCCAT);72; (14); PP.837-8, UNIV. TOKYO;FAC. PHARM. SCI.; TOKYO; JAPAN, XP002030140**
- **COBURN ET AL.: "Picrylamino-substituted Heterocycles. V. Pyridines(1,2)" JOURNAL OF HETEROCYCLIC CHEMISTRY, vol. 9, 1972, PROVO US, pages 1039-1044, XP002030141**

## Description

La présente invention concerne des matériaux à propriétés ferromagnétiques comportant des molécules organiques à propriétés magnétiques et leurs procédés de fabrication.

Les molécules constitutives des matériaux de l'invention sont dites à propriétés magnétiques, car elles présentent un moment magnétique permanent qui leur permet, notamment, de s'orienter dans un champ magnétique. Les matériaux de l'invention, qui comportent de telles molécules, sont dits ferromagnétiques, car ils présentent, en l'absence de champ extérieur, une aimantation spontanée, les moments magnétiques permanents des molécules à propriétés magnétiques qu'ils comportent tendant à s'orienter parallèlement les uns aux autres, dans le cadre d'une organisation de la phase solide.

Certains matériaux organiques sont connus pour posséder des propriétés ferromagnétiques. De tels matériaux sont décrits dans les documents brevets référencés EP-A-0 512 926, FR-A-2 710 648 et FR-A-2 684 677. Il s'agit de copolymères zwitterioniques tels que, par exemple, le copolymère suivant, formé à partir de l'aniline et de l'acide naphtylamine sulfonique :

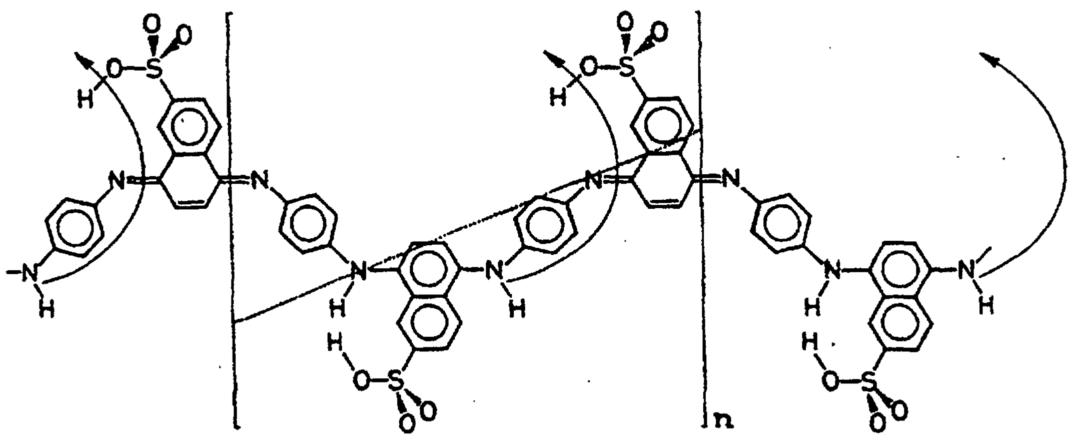

Le ferromagnétique de ce copolymère n'apparaît qu'à l'état solide, lorsqu'une torsion, stabilisée par une interaction acido-basique mettant en jeu les groupes sulfonates, se répètent tout le long de la chaîne polymérique et tout au long d'un empilement des cycles aromatiques.

Considérant l'état de la technique qui précède, un problème que se propose de résoudre l'invention est de proposer un matériau dont les propriétés ferromagnétiques dues à la présence, dans ledit matériau, de molécules organiques à propriétés magnétiques, ne soient pas dues à une torsion d'une chaîne d'un polymère ou d'un oligomère stabilisée par des interactions acido-basiques.

La solution de l'invention a pour premier objet un matériau solide à propriétés ferromagnétiques, comprenant une ou plusieurs molécules organiques à propriétés magnétiques répondant à la formule :

$$A=N=B$$

dans laquelle

- . A est un cycle quinonique, aminoquinonique ou cyclopentadiènyle substitué en positions $\alpha$ et $\alpha'$
- . B est un cycle benzénique substitué en position $\alpha'$, et
- . N est l'atome d'azote,

caractérisé en ce que A est substitué respectivement en position $\alpha$ et en position $\alpha'$ par un substituant $Y_1$, $Y_2$ accepteur d'électrons.

D'autre part, un second objet de l'invention concerne un procédé de fabrication d'un matériau solide à propriétés ferromagnétiques tel que défini ci-dessus, caractérisé en ce qu'il consiste à :

- mélanger dans un solvant un dérivé aminé $B-NH_2$ de B avec un dérivé quinonique $0=A=0$ de A ;
- à ajouter un acide de Lewis du type $BF_3$ ;

- à effectuer ledit mélange à une température entre 80° et 200°C.

Préférentiellement, le solvant est le DMSO, le mélange comporte un acide de Lewis du type $BF_3$ en quantités stoechiométriques.

La cristallisation s'effectue dans un champ magnétique.

La molécule A=N=B n'est pas macromoléculaire et ne constitue pas un polymère. Elle présente, intrinsèquement, des propriétés magnétiques ainsi que des propriétés d'organisation et de corrélation et peut, de ce fait, conduire à l'obtention d'un matériau ferromagnétique. cette molécule n'est pas stabilisée par des liaisons acido-basiques mettant en jeu des charges positives et négatives notamment des groupes sulfonates.

A= est préférentiellement un radical hydrocarboné mono ou polycyclique insaturé éventuellement polysubstitué, chaque cycle comprenant de 3 à 15 atomes de carbone et pouvant être interrompu par un ou plusieurs hétéroatomes N, S ou O.

Plus préférentiellement, A= est un cycle quinonique, aminoquinonique ou cyclopentadiènyle, au moins substitué aux positions $\alpha$ et $\alpha'$ par des substituants $Y_1$ et $Y_2$ et répondant respectivement aux formules suivantes :

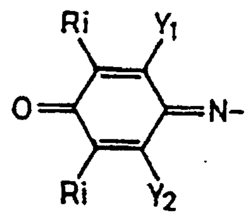 ; 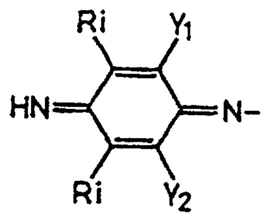 ; 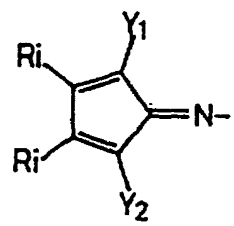 .

Bien entendu, dans le cas où A= est un radical polycyclique tel qu'un radical naphto-, anthraquinonique ou fluorényle, $Y_1$ et/ou $Y_2$ correspondent alors à une portion de cycle. Dans ce cas, des substituants répondant à la définition de $Y_1$ et/ou $Y_2$ sont alors avantageusement présents aux positions péri du radical polycyclique, par exemple, aux positions 1 et 7 dans un radical naphtoquinonique où l'atome d'azote est en position 8 ; ou aux positions 1 et 8 dans un radical anthraquinonique où l'atome d'azote est en position 9.

B- est préférentiellement un radical hydrocarboné mono ou polycyclique mono ou polysubstitué insaturé à structure conjuguée éventuellement polysubstitué, chaque cycle comprenant de 3 à 15 atomes de carbone et pouvant être interrompu par un ou plusieurs hétéroatomes N, S, ou O.

Plus préférentiellement, B- est un cycle benzénique au moins substitué aux positions $\alpha$, $\alpha'$ par des substituants $X_1$ et $X_2$ et répondant à la formule suivante :

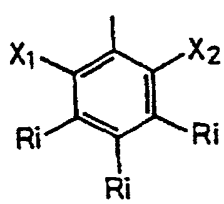

Bien entendu, dans les cas où B- est un radical polycyclique tel qu'un radical naphto ou anthracénique, $X_1$ et/ou $X_2$ correspondent alors à une portion de cycle. Dans ce cas, des substituants répondant à la définition de $X_1$ et/ou $X_2$ sont alors avantageusement présents aux positions péri du radical polycyclique, ainsi que cela est montré ci-dessous :

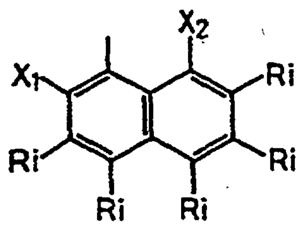
;
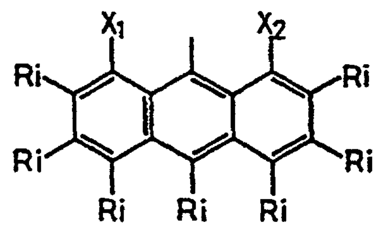

Dans les molécules définies ci-dessus pour A=N- et B-, les substituants $R_i$ sont quelconques, donneurs ou accepteurs d'électrons et sont notamment des substituants choisis parmi les radicaux définis ci-dessous pour $X_1$, $X_2$, $Y_1$ ou $Y_2$.

Les molécules de l'invention montrent un caractère magnétique plus marqué lorsque les substituants du radical cyclique de l'imine sont accepteurs d'électrons et lorsque les substituants du radical cyclique de B-sont donneurs d'électrons, c'est-à-dire lorsque lesdits substituants induisent un déplacement électronique du radical cyclique B- vers l'imine cyclique A=N- à structure conjuguée.

Aussi, dans les molécules objets de l'invention, les substituants des positions $\alpha$, $\alpha'$ du radical cyclique B-, c'est-à-dire, dans les formules précédentes, $X_1$ et $X_2$, sont avantageusement des donneurs d'électrons tels que $-NH_2$, -NRH, -NRR, -OH, -OR, -R ou -R est un radical alkyle, cycloalkyle, aryle, cycloalkylalkyle, arylalkyle et comporte éventuellement un ou plusieurs hétéroatomes. En particulier, $X_1$ et/ou $X_2$ sont choisis parmi les radicaux suivants : $-NH_2$, $-NMe_2$, -OH, -OMe, -Me et -iPr.

De plus, dans lesdites molécules, les substituants des positions $\alpha$, $\alpha'$ du cycle A= conjugué de l'imine, c'est-à-dire, dans les formules précédentes, $Y_1$ et/ou $Y_2$, sont avantageusement des accepteurs d'électrons tels que $-NO_2$, -COR, $-CO_2R$, -COOR, $-SO_3R$, $-SO_2R$ où R est un radical alkyle, cycloalkyle, aryle, cycloalkylakyle, arylalkyle et comporte éventuellement un ou plusieurs hétéroatomes. En particulier, $Y_1$ et/ou $Y_2$ sont choisis parmi les radicaux suivants : $-NO_2$, $SO_3H$, $-SO_2Me$, $-SO_3Me$, $-CO_2H$, $-CO_2Me$ et -COMe.

A l'état fondamental, chaque molécule A=N-B est sujette à des contraintes principalement de nature stérique qui leur sont imposées par les substituants qu'elles portent dans les positions $\alpha$ et $\alpha'$ de leurs radicaux cycliques A= et B-. Ces substituants encombrent ladite molécule A=N-B au niveau de l'atome d'azote et induisent alors une orthogonalisation des orbitales portant les électrons radicalaires, une rupture de la double liaison A=N entraîne la formation d'un biradical A·-·N puis le piégeage des électrons sur les cycles et lesdites molécules peuvent alors montrer une corrélation de spin caractéristique de l'état triplet. Ainsi, à l'état fondamental, les molécules de l'invention sont généralement à l'état triplet qui est à l'origine des propriétés ferromagnétiques des matériaux de l'invention. Bien entendu, l'orthogonalisation est améliorée dans le cas où les radicaux cycliques sont disubstitués aux positions $\alpha$ et $\alpha'$, ce qui augmente l'encombrement stérique au niveau de l'atome d'azote. Toutefois, on notera que les molécules A=N-B qui sont, à l'état fondamental, à l'état singulet mais dont l'énergie nécessaire au passage à l'état triplet le plus proche est faible, de l'ordre de quelques kCal/mole, peuvent facilement passer de l'état singulet à l'état triplet après une légère excitation, par exemple magnétique, chimique, électrochimique ou électrique et pourront donner lieu à une cristallisation à l'origine de matériaux à propriétés ferromagnétiques.

Dans un matériau selon l'invention, n molécules se structurent à l'état solide de manière à ce que ledit matériau présente des propriétés ferromagnétiques. n est un nombre entier supérieur ou égal à 1. n est choisi tel qu'il représente le taux de condensation à l'état solide. Cette condensation est susceptible de s'effectuer, dans l'espace, selon un axe unique, $(A=N-B)_n$ formant alors une chaîne ou selon plusieurs axes. Dans un premier exemple, la condensation des n molécules à l'état solide s'effectue par empilement moléculaire selon un premier axe selon lequel les radicaux cycliques à structure conjuguée A= sont superposés et les radicaux cycliques B- sont superposés et, dans un deuxième exemple, la condensation des n molécules à l'état solide s'effectue par empilement moléculaire selon un axe selon lequel les radicaux cycliques à structure conjuguée A= sont superposés aux radicaux cycliques B-. Dans un troisième exemple, les molécules sont empilées de la manière suivante :

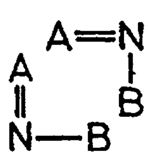
ou
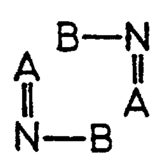

S'il n'est pas nécessaire, qu'à l'état solide cristallisé, les molécules de l'invention soient liés par des liaisons covalentes, il est particulièrement avantageux que, dans ledit état solide, lesdites molécules s'arrangent, dans l'espace, de manière ordonnée et, par exemple, s'empilent les uns sur les autres. C'est la raison pour laquelle le procédé de fabrication des matériaux de l'invention tient compte de l'étape de cristallisation. Toutefois, les molécules de l'invention peuvent être greffés le long d'un polymère non ferromagnétique ou faire l'objet d'une copolymérisation avec un ou plusieurs mono ou oligomères. Ainsi régulièrement répartis le long d'une chaîne polymérique, ces molécules forment un matériau présentant des propriétés ferromagnétiques marquées.

D'autres caractéristiques et avantages de l'invention apparaîtront à la lecture des exemples suivants, donnés à titre illustratif et nullement limitatif, et qui doivent être lus au regard des dessins annexés dans lesquels :

- les figures 1A et 1B montrent des spectres IR de matériaux selon l'invention cristallisés dans des conditions opératoires différentes définies dans un exemple 2 ;
- les figures 2A et 2B montrent des spectres RX pour les matériaux de l'exemple 2 ;
- la figure 3 représente le signal d'absorption obtenu en spectroscopie électronique RFM pour un matériau selon l'invention ;
- la figure 4 représente la variation de l'aimantation d'un matériau de l'invention en fonction du champ magnétique qui lui est appliqué ;
- les figures 5A et 5B donnent des résultats de tests d'analyse thermique différentielle/analyse thermogravitométrique obtenus pour des matériaux selon l'invention ; et
- la figure 6 montre un spectre calculé d'un fragment d'une molécule d'un matériau selon l'invention.

<u>Exemple 1</u>

Plusieurs centaines de molécules ont été examinées afin de déterminer lesquelles se trouvent, dans leur état fondamental, à l'état triplet. Ces molécules sont susceptibles de conduire à des matériaux ferromagnétiques montrant eux-mêmes un état triplet.

On a calculé, pour chacune des molécules examinées, l'énergie relative de l'état triplet par rapport à l'état singulet. Cette énergie, notée $E_{rel}$ et exprimée en eV, est négative lorsque, à l'état fondamental, une molécule se trouve dans un état triplet. Elle est positive lorsque ladite molécule est, à l'état fondamental, dans un état singulet. Un petit nombre seulement de molécules parmi l'ensemble des molécules examinées ont satisfait à ce critère. Il s'agit des molécules des types I à VI suivants, dans lesquelles $X_1$, $X_2$, $Y_1$ et $Y_2$ sont les substituants répertoriés dans les tableaux ci-après.

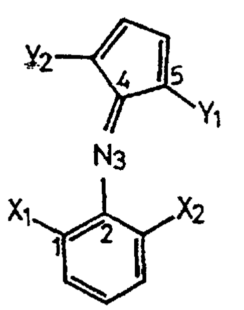
TYPE I

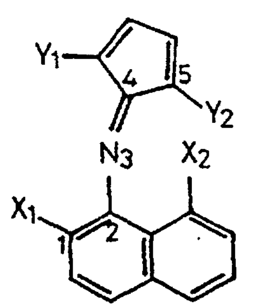
TYPE II

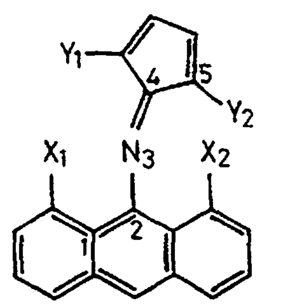
TYPE III

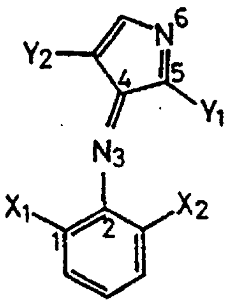

TYPE IV

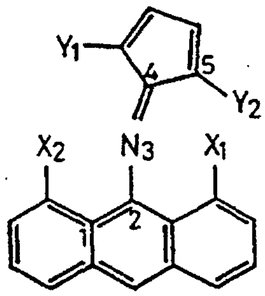

TYPE V

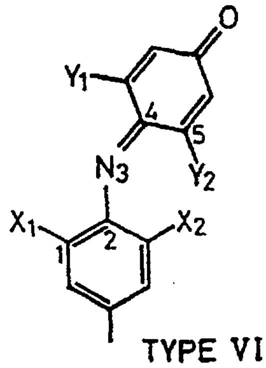

TYPE VI

| Type | I | I | I | I | I | I | I | I |
|---|---|---|---|---|---|---|---|---|
| $X_1$ | $SO_3H$ | $SO_3H$ | $SO_2Me$ | $NO_2$ | $NO_2$ | $CO_2H$ | $CO_2H$ | $CO_2Me$ |
| $X_2$ | $SO_3H$ | $SO_3H$ | $SO_2Me$ | $NO_2$ | $NO_2$ | $CO_2H$ | $CO_2H$ | $CO_2Me$ |
| $Y_1$ | $NH_2$ | $NMe_2$ | $NH_2$ | $NH_2$ | $NMe_2$ | $NH_2$ | $NMe_2$ | $NH_2$- |
| $Y_2$ | $NH_2$ | $NMe_2$ | $NH_2$ | $NH_2$ | $NMe_2$ | $NH_2$ | $NMe_2$ | $NH_2$ |
| $E_{rel}$ | -0,96 | -0,75 | -0,72 | -0,68 | -0,62 | -0,54 | -0,42 | -0,53 |
| (5,4,3,2) | 44 | 16 | 38 | 10 | 4 | 10 | 2 | 10 |
| (4,3,2,1) | -114 | -109 | -111 | -97 | -95 | -93 | -90 | -93 |
| (4,3) | 1,312 | 1,307 | 1,312 | 1,314 | 1,306 | 1,311 | 1,305 | 1,311 |
| (3,2) | 1,355 | 1,350 | 1,367 | 1,373 | 1,350 | 1,386 | 1,372 | 1,388 |
| (4,3,2) | 135,5 | 141,7 | 133,1 | 129,1 | 139,3 | 127,4 | 133,5 | 127,0 |

| Type | I | I | II | II | II | II | II | II |
|---|---|---|---|---|---|---|---|---|
| $X_1$ | $CO_2Me$ | $NH_2$ | $SO_2Me$ | $SO_2Me$ | $SO_3H$ | $SO_3H$ | $NO_2$ | $NO_2$ |
| $X_2$ | $CO_2Me$ | $NH_2$ | $SO_2Me$ | $SO_2Me$ | $SO_3H$ | $SO_3H$ | $NO_2$ | $NO_2$ |
| $Y_1$ | $NMe_2$ | $NH_2$ | $NH_2$ | $NMe_2$ | $NH_2$ | $NMe_2$ | $NMe_2$ | $NH_2$ |
| $Y_2$ | $NMe_2$ | $NH_2$ | $NH_2$ | $NMe_2$ | $NH_2$ | $NMe_2$ | $NMe_2$ | $NH_2$ |
| $E_{rel}$ | -0,43 | -0,50 | -0,80 | -0,81 | -0,86 | -0,80 | -0,82 | -0,63 |
| (5,4,3,2) | 1 | 2 | -92 | -82 | -104 | -99 | -158 | -166 |
| (4,3,2,1) | -89 | -88 | 0 | -11 | 19 | 2 | 46 | 34 |
| (4,3) | 1,305 | 1,316 | 1,321 | 1,324 | 1,319 | 1,327 | 1,323 | 1,318 |
| (3,2) | 1,373 | 1,412 | 1,315 | 1,317 | 1,322 | 1,325 | 1,361 | 1,350 |
| (4,3,2) | 133,6 | 121,8 | 139,5 | 139,7 | 141,4 | 138,7 | 129,6 | 131,8 |

| Type | II | II | III | III | III | III | III | III |
|---|---|---|---|---|---|---|---|---|
| $X_1$ | $CO_2H$ | $CO_2H$ | $NH_2$ | $NH_2$ | $SO_3H$ | COOH | $SO_3H$ | $NO_2$ |
| $X_2$ | $CO_2H$ | $CO_2H$ | $NH_2$ | $NH_2$ | $SO_3H$ | COOH | $SO_3H$ | $NO_2$ |
| $Y_1$ | $NH_2$ | $NMe_2$ | COOH | $NO_2$ | $NH_2$ | $NH_2$ | $NMe_2$ | $NH_2$ |
| $Y_2$ | $NH_2$ | $NMe_2$ | COOH | $NO_2$ | $NH_2$ | $NH_2$ | $NMe_2$ | $NH_2$ |

(suite)

| Type | II | II | III | III | III | III | III | III |
|---|---|---|---|---|---|---|---|---|
| $E_{rel}$ | -0,58 | -0,36 | -0,02 | -0,02 | -0,13 | -0,50 | -0,03 | -0,29 |
| (5,4,3,2) | -175 | - | 85 | 83 | 23 | 17 | 51 | 15 |
| (4,3,2,1) | 86 | - | -1 | 2 | 13 | 34 | 14 | 23 |
| (4,3) | 1,315 | - | 1,333 | 1,319 | 1,344 | 1,323 | 1,391 | 1,323 |
| (3,2) | 1,394 | - | 1,290 | 1,293 | 1,316 | 1,352 | 1,306 | 1,335 |
| (4,3,2) | 125,5 | - | 140,3 | 143,6 | 130,3 | 131,5 | 123,3 | 133,3 |

| Type | III | IV | IV | IV | V | V | VI |
|---|---|---|---|---|---|---|---|
| $X_1$ | $CO_2H$ | $SO_3H$ | $SO_3H$ | $NO_2$ | $NH_2$ | $NH_2$ | $NH_2$ |
| $X_2$ | $CO_2H$ | $SO_3H$ | $SO_3H$ | $NO_2$ | $NH_2$ | $NH_2$ | $NH_2$ |
| $Y_1$ | $NMe_2$ | $NH_2$ | $NMe_2$ | $NMe_2$ | COOH | $NO_2$ | $SO_3H$ |
| $Y_2$ | $NMe_2$ | $NH_2$ | $NMe_2$ | $NMe_2$ | COOH | $NO_2$ | $SO_3H$ |
| $E_{rel}$ | -0,40 | -1,03 | -0,93 | -0,64 | -0,02 | -0,02 | -0,05 |
| (5,4,3,2) | -2 | 45 | 43 | 8 | 85 | 83 | -73 |
| (4,3,2,1) | 90 | -115 | -135 | -91 | -1 | 2 | 145 |
| (4,3) | 1,310 | 1,308 | 1,308 | 1,305 | 1,333 | 1,319 | 1,342 |
| (3,2) | 1,389 | 1,354 | 1,338 | 1,367 | 1,290 | 1,293 | 1,328 |
| (4,3,2) | 129,4 | 135,9 | 141,1 | 133,2 | 140,3 | 143,6 | 138,3 |

Dans ces tableaux, (5,4,3,2) représente l'angle dièdre ($C_5$,$C_4$,$N_3$,$C_2$) exprimé en degrés, (4,3,2,1) représente l'angle dièdre ($C_4$,$N_3$,$C_2$,$C_1$) exprimé en degrés, (4,3) représente la distance ($C_4$,$N_3$) exprimée en Å, (4,3,2) représente l'angle ($C_4$,$N_3$,$C_2$) exprimé en degrés et (3,2) représente la distance ($N_3$,$C_2$) exprimée en Å. Les valeurs de (5,4,3,2) et (4,3,2,1) permettent de connaître la situation relative des deux noyaux cycliques et (4,3,2) témoigne des contraintes, notamment stériques, qui s'exercent sur l'atome d'azote.

On déduit des tableaux ci-dessus que les molécules à l'état triplet dans leur état fondamental présentent un angle (4,3,2), compris entre environ 120° et environ 145°, qui varie de 123,3 à 143,6 est en moyenne égal à environ 130°. Les orbitales portées par les molécules sont de ce fait quasi-orthogonales.

Ces tableaux montrent par ailleurs que les substituants $X_1$, $X_2$, $Y_1$, $Y_2$ agissent non seulement par leur qualité de donneur ou d'accepteur d'électrons, mais aussi par l'encombrement stérique qu'ils provoquent sur l'atome d'azote. Ainsi, l'énergie $E_{rel}$ est plus faible lorsque $X_1 = X_2 = -NH_2$ que lorsque $X_1 = X_2 = -NMe_2$ même si $-NH_2$ est un moins bon donneur d'électrons que $-NMe_2$, $-NH_2$ apportant certainement un meilleur compromis eu égard à l'encombrement stérique qu'il apporte sur l'atome d'azote.

Bien entendu, l'énergie relative calculée de l'état triplet est valable pour des molécules considérées à l'état gazeux. A l'état solide, ces résultats peuvent être modifiés.

Exemple 2

Différentes voies de synthèse des matériaux de l'invention ont été envisagées. Dans le présent exemple, on a mélangé sous agitation, dans un ballon bicol de 100 ml, 250 mg (1,49 mmol) de 2,3,5,6-tétraaminobenzoquinone et 600 mg (3,39 mmol) de 2,6-diisopropylaniline dans 6,5 ml de DMSO (diméthylsulfoxide). On a alors lentement ajouté 1 ml de $BF_3$,$Et_2O$ et le mélange obtenu a été chauffé à 140°C pendant 12 heures. Puis, après avoir stoppé l'agitation, on a laissé le mélange refroidir lentement jusqu'à la température ambiante. Quelques heures plus tard, le mélange a été filtré sur fritté N° 4 et l'on a obtenu un matériau solide de couleur noire que l'on a lavé plusieurs fois à l'éthanol et séché sous vide. Ce matériau, obtenu à partir de la réaction ci-dessous, contiendrait un mélange des molécules I et II suivantes

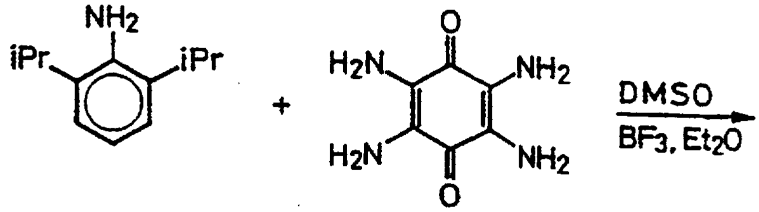


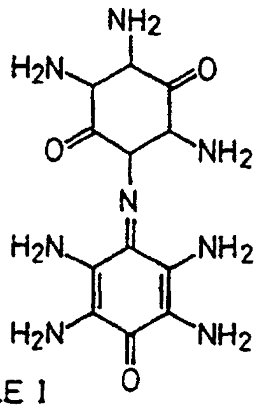

MOLECULE I

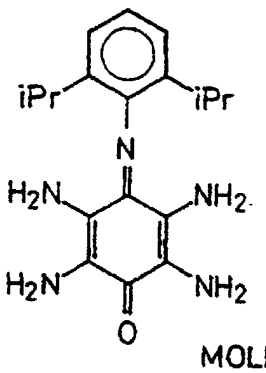

MOLECULE II

Il est toutefois possible que ledit matériau comporte en outre des molécules comportant plus de deux cycles, notamment trois ou soient des dimères des molécules I et II.

12,5 mg de ce matériau, ont été placés sur une balance de Faraday qui montre une force magnétique dont la valeur est de 17,36 contre 1380 pour la même masse de nickel en poudre. Il est donc ferromagnétique. Sachant que le nickel possède une aimantation à saturation de 40 unités électro-magnétiques (uem)/g, on en déduit que le produit synthétisé possède une aimantation voisine de 0,5 uem/g.

L'addition de quantités de plus en plus importantes de $BF_3$ dans le milieu réactionnel ne permet pas l'obtention de quantités plus importantes de produit ferromagnétique. D'ailleurs, les deux essais suivants, effectués dans les conditions de synthèse précitées du présent exemple, montrent qu'une augmentation de la quantité de $BF_3$ réduit le caractère ferromagnétique des matériaux de l'invention.

| Essai | Quantité de $BF_3$ (ml) | Force magnétique pour 12,5 mg de produit |
|---|---|---|
| 1 | 1,0 | 17,36 |
| 2 | 3,0 | 0,85 |

Toutefois, et ainsi que cela est montré aux figures 1A et 1B, les spectres IR des produits des essais 1 et 2 sont semblables. Il semble de ce fait qu'un même matériau montre des propriétés ferromagnétiques marquées ou non selon les conditions de cristallisation qui lui ont été imposées. Toutefois, ainsi que le montre la figure 2A, les pics du spectre RX du matériau du premier essai, qui correspondent au matériau dont les propriétés ferromagnétiques sont marquées, sont mieux définis et plus fins que les pics qui correspondent au matériau du second essai. Le matériau ferromagnétique du premier essai présente donc un ordre moléculaire plus important que celui du second essai.

La cristallisation de molécules selon l'invention dans du DMSO apparaîtrait particulièrement avantageuse pour l'obtention de matériaux à propriétés ferromagnétiques marquées.

De manière à montrer que la cristallisation constitue une étape importante dans l'obtention de matériaux à propriétés ferromagnétiques marquées, on a solubilisé le composé du second essai dans du DMSO à des températures de l'ordre de 140 °C et pendant 12 heures et on a ensuite laissé refroidir le mélange jusqu'à la température ambiante. La mesure de la force magnétique de ce matériau sur une balance de Faraday révèle que l'aimantation est modifiée dans le sens de l'augmentation.

Dans le même but, le mélange réactionnel a été, dans un premier essai, filtré puis le filtrat obtenu a été lavé à l'éthanol. Dans un second essai, ledit mélange a dans un premier temps, été évaporé le DMSO et obtenu alors une huile noire à laquelle on a ajouté du dichlorométhane, ce qui a permis, dans un second temps, de précipiter un produit

qui a été récupéré par filtration.

| Essai | Traitement | Force magnétique pour 31,6 mg de produit |
|---|---|---|
| 1 | filtration | 22,94 |
| 2 | évaporation | 0,37 |

On voit que l'aimantation dépend du traitement de précipitation. On peut donc affirmer que l'aimantation est bien attachée aux chaînes organiques et à l'ordre en phase solide.

Exemple 3 :

500 mg de 2,3,5,6-tétraaminobenzoquinone ont été dissous dans 8 ml de DMSO en présence de 1 ml de $BF_3$ éthérate, puis le mélange obtenu a été chauffé à 136°C pendant 12 heures. Le produit noir qui a été obtenu, n'est pas, contrairement à ce à quoi l'on aurait pu s'attendre, ferromagnétique, même si le spectre infra-rouge du composé est semblable à celui du matériau ferromagnétique.

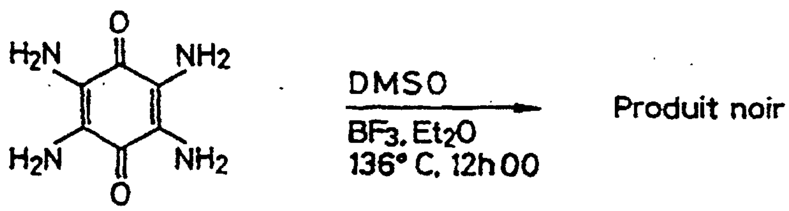


D'autres synthèses effectuées en présence de DMSO et en présence de $BF_3$ éthérate à 136°C et pendant 12 heures n'ont pas permis d'obtenir des matériaux ferromagnétiques de l'invention. Par exemple, les réactions avec la tétraphénylcyclopentadiénone n'ont pas donné les résultats attendus que ce soit avec la diisopropylaniline ou la triméthylaniline étant donné que l'on a récupéré, dans les deux cas, un produit de départ inchangé.

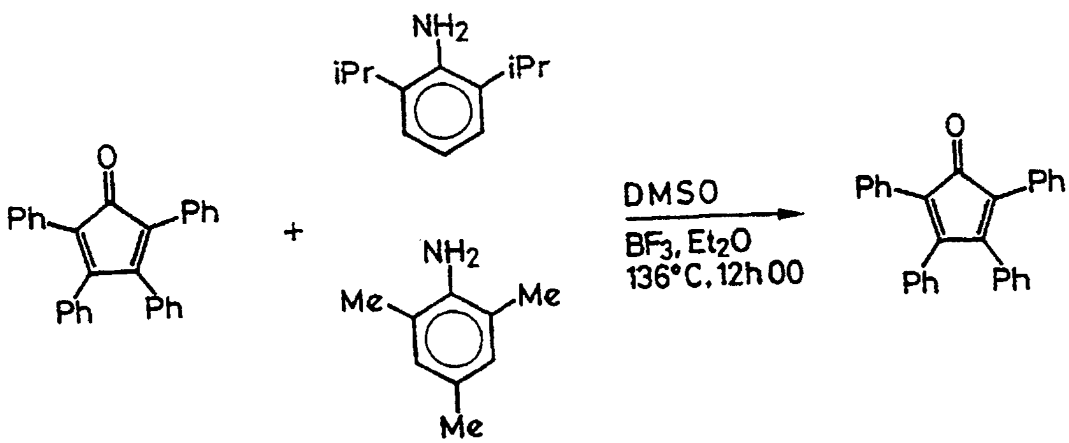


Exemple 4 :

Dans cet exemple, le matériau à propriétés ferromagnétiques marquées de l'exemple 2 a tout d'abord été testé en RMN. Ce test a permis de constater que les signaux de résonnance des noyaux sont fortement perturbés par le champ interne.

Ce matériau a ensuite été testé en spectroscopie électronique RFM. Le signal obtenu, montré en figure 3, est un signal sinusoïdal de résonnance élargi s'étendant entre 0 et 7000 Gauss, qui correspond à un matériau ferro ou ferrimagnétique présentant une forte corrélation entre les spins.

Enfin, un dernier test effectué dans un magnétomètre à gradient de champ alternatif a permis de réaliser une mesure absolue d'aimantation du matériau à une température de 300 K. En mesurant la variation du signal magnétique M en fonction de la variation cyclique du champ H, on obtient la courbe de la figure 4, dans laquelle le champ magnétique H, donné en Oersted (Oe), est placé en abscisse et le signal magnétique M ou aimantation, en uem, est donné en

ordonnée. Cette courbe est caractéristique d'un composé ferromagnétique. Elle montre que l'aimantation du matériau à saturation est voisine de 0,5 uem/g à 1 % près, soit environ 1/100 de l'oxyde de fer. L'aimantation maximale est atteinte à 10 % près à 9.600 Oe et elle est complète à 16.000 Oe. Le champ coercitif est donc faible et l'hystérésis nulle ou presque, inférieure ou égale à 100 Oe.

Les trois tests du présent exemple confirment donc que le matériau de l'invention possède des propriétés ferromagnétiques et présente, dans sa phase solide, une haute corrélation entre les spins.

Exemple 5

On effectue sur le matériau à propriétés ferromagnétiques marquées de l'exemple 2 une analyse quantitative des teneurs en Fe, Ni, Co. L'ensemble de ces mesures donnent des teneurs très largement inférieures à 100 ppm, ce qui confirme que le niveau d'aimantation observé est bien d'origine organique.

Exemple 6 :

Le matériau à propriétés ferromagnétiques marquées de l'exemple 2 est mis en présence d'une vapeur de métacrésol connue en tant que dopant secondaire, notamment des polyalinines, en augmentant leur conductivité. Ce dopage réduit l'aimantation des composés ferromagnétiques à base d'aniline et d'acide naphtalène amino sulfonique. Il en est de même dans le cas présent. L'aimantation disparaît en présence de telles vapeurs, ce qui confirme le caractère organique.

Exemple 7 :

Le matériau à propriétés ferromagnétiques marquées de l'exemple 2 est dissous dans du DMSO puis recristallisé au moyen de dichlorométhane dans un champ magnétique de 2 teslas généré par un électroaimant. Alors que le matériau possède, dans son état initial, une aimantation de 0,5 uem/g, l'aimantation progresse jusqu'à 5 uem/g après cristallisation.

Exemple 8 :

Afin de vérifier la stabilité thermique des matériaux à propriétés ferromagnétiques de l'invention, on a effectué des analyses thermiques différentielles (ATD) et thermogravitométriques (ATG) sur les matériaux obtenus dans les essais 1 et 2 de l'exemple 2 séchés sous vide. Les résultats de ces analyses sont respectivement présentés aux figures 5A et 5B dans lesquelles l'échelle des abscisses représente la température en degrés Celsius, l'échelle des ordonnées de gauche représente la perte de poids TG en % et l'échelle des ordonnées de droite représente la température différentielle DTA.

Les résultats de ces essais montrent que les matériaux de l'invention ont une remarquable stabilité thermique à l'air puisqu'il est possible de les chauffer jusqu'à des températures de l'ordre de 200°C sans que leurs propriétés ferromagnétiques soient affectées.

Exemple 9 :

Le matériau à propriétés ferromagnétiques marquées de l'exemple 2 a été placé dans une bobine de fil métallique. Le champ magnétique généré à l'intérieur de la bobine s'en est trouvé modifié.

Exemple 10 :

Afin de confirmer les résultats de spectroscopie infra-rouge présentés aux figures 1A et 1B, des calculs ab initio ont été menés, dans lesquels le spectre du fragment de molécule suivant :

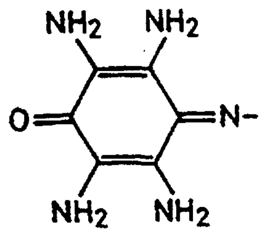

a été modélisé. Ce spectre calculé est défini par des valeurs consignées dans les tableaux suivants, dans lesquels a. correspond à l'absorbance et n.o. correspond au nombre d'onde en $cm^{-1}$ :

| | | | | | |
|---|---|---|---|---|---|
| a. | 0,0977 | 0,1025 | 0,1082 | 0,1244 | 0,1324 |
| n.o. | 502,2 | 526,7 | 556,3 | 639,5 | 680,8 |
| a. | 0,1361 | 0,1368 | 0,1420 | 0,1511 | 0,1544 |
| n.o. | 699,7 | 718,4 | 729,9 | 766,7 | 793,6 |
| a. | 0,1613 | 0,1652 | 0,1685 | 0,1852 | 0,1952 |
| n.o. | 829,2 | 849,4 | 865,9 | 952,2 | 100,3 |
| a. | 0,1981 | 0,2114 | 0,2166 | 0,2265 | 0,2292 |
| n.o. | 1018,1 | 1086,6 | 1113,5 | 1164,3 | 1178,2 |
| a. | 0,2387 | 0,2667 | 0,2750 | 0,2921 | 0,2954 |
| n.o. | 1226,8 | 1371,0 | 1413,7 | 1501,7 | 1518,3 |
| a. | 0,2997 | 0,3051 | 0,3056 | 0,3116 | 0,3134 |
| n.o. | 1540,6 | 1568,6 | 1571,1 | 1601,8 | 1610,8 |
| a. | 0,3192 | 0,6494 | 0,6512 | 0,6531 | 0,6534 |
| n.o. | 1641,0 | 3338,4 | 3347,4 | 3357,4 | 3558,7 |
| a. | 0,6653 | 0,6727 | 0,6732 | 0,6735 | 0,6750 |
| n.o. | 3420,0 | 3457,9 | 3460,6 | 3462,3 | 3470,0 |

n.o. 3420,0 3457,9 3460,6 3462,3 3470,0

Le spectre modélisé du fragment moléculaire précité, représenté à la figure 6 se superpose bien aux spectres IR des figures 1A et 1B, ce qui confirme que les molécules des matériaux de l'invention obtenus dans l'exemple 2 comportent un tel fragment.

Les matériaux à propriétés ferromagnétiques de l'invention peuvent être déposés en couche mince dont l'épaisseur peut être extrêmement faible, de l'ordre du nanomètre, sur des supports tels que le polyéthylène, le polychlorure de vinyle, le polyméthacrylate de méthyle, des polycarbonates et des résines époxydes. De tels dépôts en couche mince peuvent être effectués entre des couches dont les propriétés sont différentes. Ces couches sont notamment choisies parmi des couches diamagnétiques, ferromagnétiques, ferrimagnétiques, et paramagnétiques.

Par ailleurs, un ajustement des propriétés magnétiques des matériaux de l'invention est possible. Cet ajustement peut être réalisé par voie chimique à l'aide d'une structure catalytique, par voie électrochimique, par voie mécanique au moyen de la pression, notamment à l'aide d'un matériau piézoélectrique, ou par voie thermique.

Déposés en couches minces ou non, les matériaux à propriétés ferromagnétiques de l'invention, montrant, le cas échéant, des propriétés ferromagnétiques ajustées, sont susceptibles d'être utilisés dans un grand nombre de domaines.

Des exemples non limitatifs d'utilisations des matériaux de l'invention sont donnés ci-dessous. Il s'agit d'applications dans les domaines des hyperfréquences, des dispositifs magnétiques en général, des dispositifs électrotechniques, des dispositifs de stockage de l'information et du domaine de la photonique, dans le domaine de la médecine et dans la fabrication de conducteurs ou supraconducteurs. Dans le domaine des hyperfréquences, les matériaux de l'invention sont susceptibles d'être utilisés dans des déphaseurs à ferrites et antennes ferritiques, des circulateurs et isolateurs et blindages électromagnétiques, des tubes à ondes progressives et klystrons, des dispositifs d'inductance, des lignes à retard, des puces hyperfréquences. Dans le domaine des dispositifs magnétiques, les matériaux de l'invention sont susceptibles d'être utilisés dans des bandes et cartes magnétiques, comme substrat pour mémoires à bulles, dans des dispositifs de téléphonie tels que des capteurs, actuateurs et amplificateurs, dans des relais magnétiques, dans des amortisseurs et freins notamment à courants de Foucault, pour la fabrication de fluides magnétorhéologiques, dans des compas et dans des dispositifs magnétiques de contrôle et de sécurité tels que des thermostats, des jauges. Dans le domaine de l'électrotechnique, les matériaux de l'invention sont susceptibles d'être utilisés dans des noyaux ferritiques, des systèmes de lévitation et paliers, des têtes d'écriture et d'effacement, des moteurs avec ou sans contact et des transformateurs. Dans le domaine du stockage de l'information et de la photonique, les dispositifs de l'invention sont susceptibles d'être utilisés dans des mémoires magnétorésistives, des mémoire à effet Hall, des mémoires optiques réinscriptibles , des dispositifs d'imagerie magnétique et d'holographie, des déphaseurs et convertisseurs. Enfin, dans le domaine de la médecine, les matériaux de l'invention sont susceptibles d'être utilisés dans des dispositifs d'imagerie de la médecine nucléaire, dans des médicaments utiles dans des traitements de pathologies d'origine virale et/ou bactérienne.

## Revendications

**1.** Matériau solide à propriétés ferromagnétiques, comportant une ou plusieurs molécules organiques à propriétés magnétiques répondant à la formule :

$$A=N-B$$

dans laquelle

- . A est un cycle quinonique, aminoquinonique ou cyclopentadiènyle substitué en positions $\alpha$ et $\alpha'$
- . B est un cycle benzénique substitué en position $\alpha'$, et
- . N est l'atome d'azote,

**caractérisé en ce que** A est substitué respectivement en position $\alpha$ et en position $\alpha'$ par un substituant $Y_1$, $Y_2$ accepteur d'électrons.

**2.** Matériau selon la revendication 1, **caractérisé en ce que** $Y_1$ et $Y_2$ sont choisis parmi les radicaux suivants : $-NO_2$, -COR, - $CO_2R$, -CORR, $-SO_3R$, $-SO_2R$ où R est radical alkyle, cycloalkyle, aryle, cycloalkylalkyle, arylalkyle et, comporte éventuellement un ou plusieurs hétéroatomes.

**3.** Matériau selon la revendication 2, **caractérisé en ce que** $Y_1$ et $Y_2$ sont choisis parmi les radicaux suivants : $-NO_2$, $-SO_3H$, $-SO_2Me$, $-CO_2H$, $-CO_2Me$, -COMe.

**4.** Matériau selon la revendication 1, **caractérisé en ce que** le radical B est substitué, en position $\alpha$, par un substituant $X_1$ donneur d'électrons.

**5.** Matériau selon la revendication 4, **caractérisé en ce que** le radical B est substitué, en position $\alpha'$, par un substituant $X_2$ donneur d'électrons.

**6.** Matériau selon l'une quelconque des revendications 4 ou 5, **caractérisé en ce que** $X_1$ et/ou $X_2$ sont choisis parmi les radicaux suivants :
-X où X est un atome d'halogène, $-NH_2$, -NRH, -NRR, -OH, -OR, -R où R est un radical alkyle, cycloalkyle, aryle, cycloalkylalkyle, arylalkyle et, comporte éventuellement un ou plusieurs hétéroatomes.

**7.** Matériau selon la revendication 6, **caractérisé en ce que** X1 et/ou X2 sont choisis parmi les radicaux suivants : $-NH_2$, $-NMe_2$, -OH, -OMe, -Me et iPr.

**8.** Matériau selon l'une des revendications précédentes, **caractérisé en ce qu'**il comporte une ou plusieurs molécules

répondant à l'une des formules suivantes :

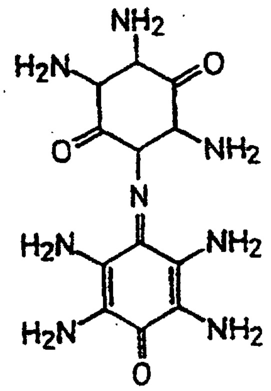

ou

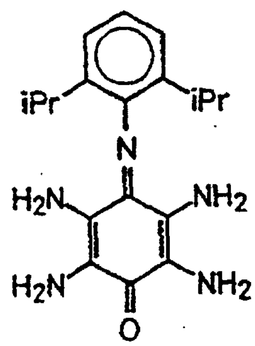

**9.** Matériau solide à propriétés ferromagnétiques, **caractérisé en ce qu'**il comporte n molécules selon l'une des revendications 1 à 7, n étant un nombre entier supérieur ou égal à 2 et **en ce que** les n molécules font l'objet d'une condensation, le matériau répondant à la formule suivante $(A{=}N{-}B)_{n'}$ dans laquelle n est le taux de condensation.

**10.** Matériau selon la revendication 9, **caractérisé en ce que** les n molécules sont liées par des liaisons covalentes.

**11.** Matériau selon l'une des revendications précédentes, **caractérisé en ce que** les molécules sont greffées le long d'un polymère.

**12.** Matériau selon l'une des revendications précédentes, **caractérisé en ce que** les molécules font l'objet d'une copolymérisation avec un ou plusieurs mono ou oligomères.

**13.** Procédé de fabrication d'un matériau selon l'une des revendications 1 à 12, **caractérisé en ce qu'**il consiste à :

- mélanger dans un solvant un dérivé aminé $B{-}NH_2$ de B avec un dérivé quinonique 0=A=0 de A ;
- à ajouter un acide de Lewis du type $BF_3$ ;
- à effectuer ledit mélange à une température entre 80° et 200°C.

**14.** Procédé selon la revendication 13, **caractérisé en ce que** le solvant est le DMSO.

**15.** Procédé selon la revendication 13, **caractérisé en ce que** l'acide de Lewis est ajouté en quantités stoechiométriques.

**16.** procédé selon l'une des revendications 13 à 15, **caractérisé en ce que** on cristallise le matériau dans un champ magnétique.

**Patentansprüche**

**1.** Material mit ferromagnetischen Eigenschaften, das ein oder mehrere organische Moleküle mit magnetischen Eigenschaften gemäss der folgenden Formel umfasst:

A=N=B

bei der

. A ein in den Positionen $\alpha$ und $\alpha'$ substituierter chinoider, aminochinoider oder cyclopentadienyler Zyklus ist,
. B ein in der Position $\alpha'$ substituierter benzolischer Zyklus ist, und
. N das Stickstoffatom ist,

**dadurch gekennzeichnet, dass** A jeweils in Position α und in Position α' von einem Elektronen akzeptierenden Substituenten $Y_1$, $Y_2$ substituiert wird.

2. Material nach Anspruch 1, **dadurch gekennzeichnet, dass** $Y_1$ und $Y_2$ unter den folgenden Radikalen gewählt werden: $-NO_2$, -COR, $-CO_2R$, -CORR, $-SO_3R$, $-SO_3R$, $-SO_2R$ wobei R ein ein Alkyl-, Cycloalkyl-, Aryl-, Cycloalkylalkyl-, Arylalkyl-Radikal ist und eventuell ein oder mehrere Heteroatome umfasst.

3. Material nach Anspruch 2, **dadurch gekennzeichnet, dass** $Y_1$ und $Y_2$ unter den folgenden Radikalen gewählt werden: $-NO_2$, $SO_3H$, $-SO_2Me$, $-CO_2H$, $-CO_2Me$ und -COMe.

4. Material nach Anspruch 1, **dadurch gekennzeichnet, dass** das Radikal B in Position α von einem Elektronen gebenden Substituenten $X_1$ substituiert wird.

5. Material nach Anspruch 1, **dadurch gekennzeichnet, dass** das Radikal B in Position α' von einem Elektronen gebenden Substituenten $X_2$ substituiert wird.

6. Material nach einem der Ansprüche 4 oder 5, **dadurch gekennzeichnet, dass** $X_1$ und/oder $X_2$ unter den folgenden Radikalen gewählt werden: -X, wobei X ein Halogenatom ist, $-NH_2$, -NRH, -NRR, -OH, -OR, -R, wobei R ein Alkyl-, Cycloalkyl-, Aryl-, Cycloalkylalkyl-, Arylalkyl-Radikal ist und eventuell ein oder mehrere Heteroatome umfasst.

7. Material nach Anspruch 6, **dadurch gekennzeichnet, dass** $X_1$ und/oder $X_2$ unter den folgenden Radikalen gewählt werden: $-NH_2$, $-NMe_2$, -OH, -OMe, -Me und -iPr.

8. Material nach einem der vorangehenden Ansprüche, **dadurch gekennzeichnet, dass** es ein oder mehrere Moleküle gemäss einer der folgenden Formeln umfasst:

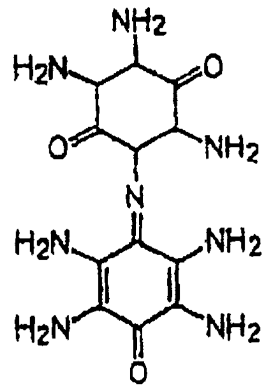

oder

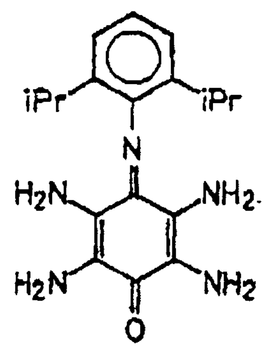

9. Solides Material mit ferromagnetischen Eigenschaften, **dadurch gekennzeichnet, dass** es n Moleküle nach einem der Ansprüche 1 bis 7 umfasst, wobei n eine Ganzzahl von über oder gleich 2 ist, und dass n Moleküle Gegenstand einer Kondensierung sind, wobei das Material der folgenden Formel entspricht (A=N-B)n', bei der $n_1$ die Kondensierungsrate ist.

10. Material nach Anspruch 9, **dadurch gekennzeichnet, dass** die n Moleküle durch kovalente Verbindungen verbunden sind.

11. Material nach einem der vorangehenden Ansprüche, **dadurch gekennzeichnet, dass** die Moleküle entlang einem Polymehr eingepflanzt sind.

12. Material nach einem der vorangehenden Ansprüche, **dadurch gekennzeichnet, dass** die Moleküle Gegenstand einer Kopolymerisierung mit einem oder mehreren Mono- oder Oligomeren sind.

13. Herstellungsverfahren eines Materials nach einem der Ansprüche 1 bis 12, **dadurch gekennzeichnet, dass** es darin besteht:

- in einem Lösemittel ein Aminderivat $B-NH_2$ von B mit einem chinoiden Derivat 0=A=0 von A zu mischen;
- eine Lewis-Säure des Typs $BF_3$ hinzuzufügen,
- das besagte Mischen bei einer Temperatur zwischen 80° und 200°C durchzuführen.

**14.** Verfahren nach Anspruch 13, **dadurch gekennzeichnet, dass** das Lösemittel DMSO ist.

**15.** Verfahren nach Anspruch 13, **dadurch gekennzeichnet, dass** die Lewis-Säure in stöchiometrischen Mengen beigefügt wird.

**16.** Verfahren nach einem der Ansprüche 13 bis 15, **dadurch gekennzeichnet, dass** man das Material in einem Magnetfeld kristallisiert.

## Claims

**1.** A solid material with ferromagnetic properties, having one or more organic molecules with magnetic properties complying with the formula:

A=N-B

in which

- A is a quinonic, aminoquinonic or cyclopentadiene ring substituted in positions $\alpha$ and $\alpha'$
- B is a benzene ring substituted in position $\alpha$, and
- N is the nitrogen atom,

**characterised in that** A is substituted respectively in position $\alpha$ and in position $\alpha'$ by an electron-accepting substituent $Y_1$, $Y_2$.

**2.** A material according to Claim 1, **characterised in that** $Y_1$ and $Y_2$ are chosen from amongst the following radicals: $-NO_2$, -COR, $-CO_2R$, -CORR, $-SO_3R$ or $-SO_2R$ where R is an alkyl, cycloalkyl, aryl, cycloalkylalkyl or arylalkyl radical and possibly has one or more heteroatoms.

**3.** A material according to Claim 2, **characterised in that** $Y_1$ and $Y_2$ are chosen from amongst the following radicals: $-NO_2$, $-SO_3H$, $-SO_2Me$, $-CO_2H$, $-CO_2Me$ or -COMe.

**4.** A material according to Claim 1, **characterised in that** the radical B is substituted, in position $\alpha$, by a substituent $X_1$ which is an electron donor.

**5.** A material according to Claim 4, **characterised in that** the radical B is substituted, in position $\alpha'$, by a substituent $X_2$ which is an electron donor.

**6.** A material according to either one of Claims 4 or 5, **characterised in that** $X_1$ and/or $X_2$ are chosen from amongst the following radicals:

- - X where X is a halogen atom, $-NH_2$, -NRH, -NRR, -OH, -OR, -R where R is an alkyl, cycloalkyl, aryl, cycloalkylalkyl or arylalkyl radical and possibly has one or more heteroatoms.

**7.** A material according to Claim 6, **characterised in that** $X_1$ and/or $X_2$ are chosen from amongst the following radicals: $-NH_2$, $-NMe_2$, -OH, -OMe, -Me and iPr.

**8.** A material according to one of the preceding claims, **characterised in that** it has one or more molecules complying with one of the following formulae:

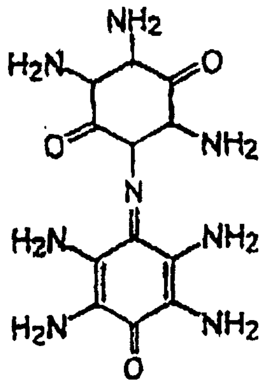

ou

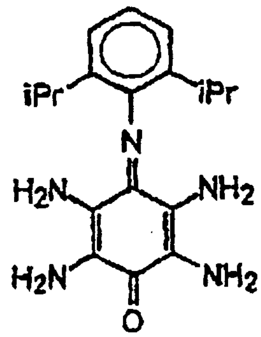

9. A solid material with ferromagnetic properties, **characterised in that** it has n molecules according to one of Claims 1 to 7, n being an integer number greater than or equal to 2, and **in that** the n molecules are the subject of condensation, the material complying with the following formula $(A{=}N{-}B)_n$ in which n is the condensation rate.

10. A material according to Claim 9, **characterised in that** the n molecules are connected by covalent bonds.

11. A material according to one of the preceding claims, **characterised in that** the molecules are grafted along a polymer.

12. A material according to one of the preceding claims, **characterised in that** the molecules are the subject of copolymerisation with one or more mono or oligomers.

13. A method of manufacturing a material according to one of Claims 1 to 12, **characterised in that** it consists of:

- mixing in a solvent an amine derivative $B{-}NH_2$ of B with a quinone derivative 0=A=0 of A;
- adding a Lewis acid of the $BF_3$ type;
- effecting the said mixing at a temperature of between 80° and 200°C.

14. A method according to Claim 13, **characterised in that** the solvent is DMSO.

15. A method according to Claim 13, **characterised in that** the Lewis acid is added in stoichiometric quantities.

16. A method according to one of Claims 13 to 15, **characterised in that** the material is crystallised in a magnetic field.

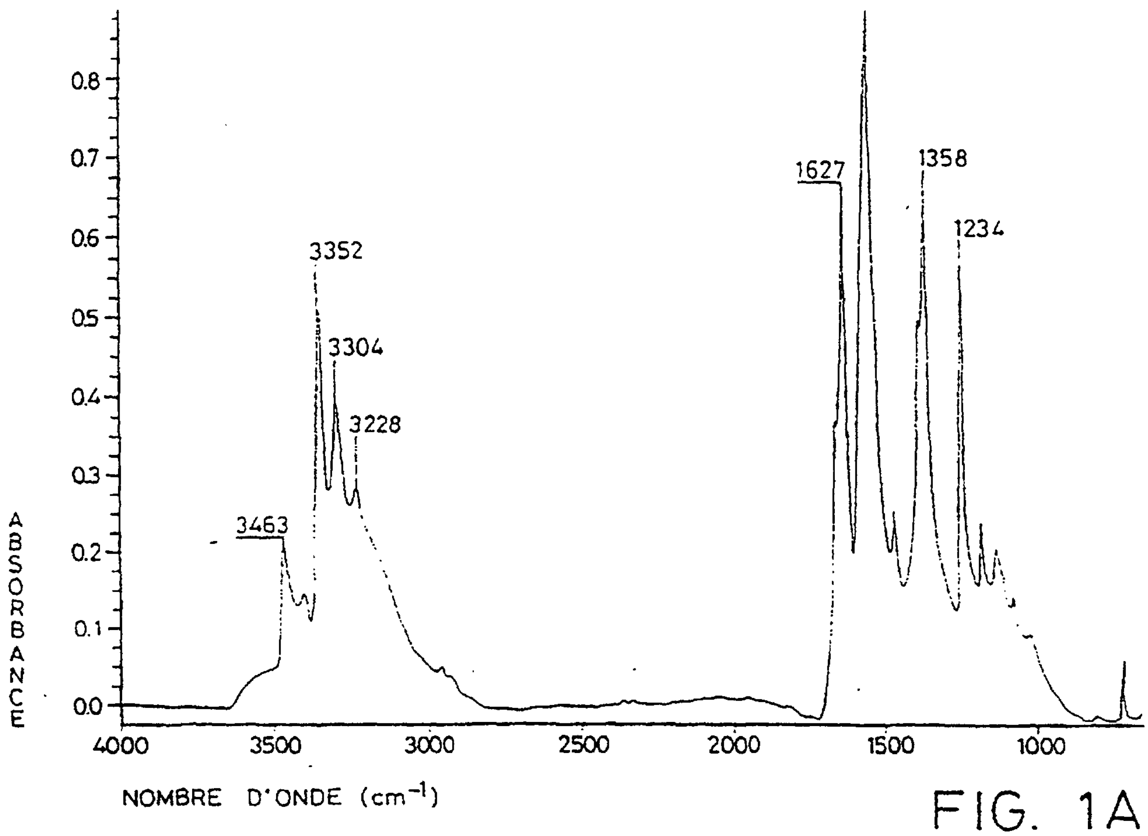
ABSORBANCE
0.8
0.7
0.6
0.5
0.4
0.3
0.2
0.1
0.0
3463
3352
3304
3228
1627
1358
1234
4000
3500
3000
2500
2000
1500
1000
NOMBRE D'ONDE (cm-1)

FIG. 1A

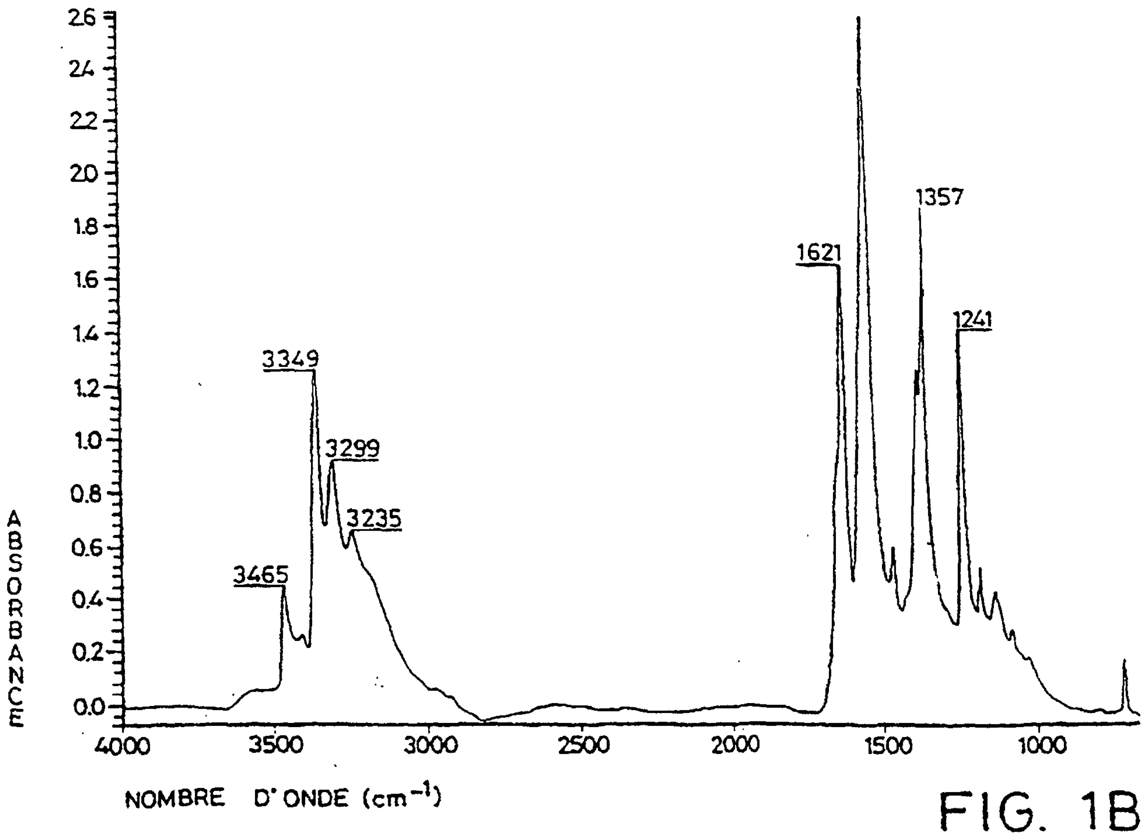
ABSORBANCE
2.6
2.4
2.2
2.0
1.8
1.6
1.4
1.2
1.0
0.8
0.6
0.4
0.2
0.0
3465
3349
3299
3235
1621
1357
1241
4000
3500
3000
2500
2000
1500
1000
NOMBRE D'ONDE (cm-1)

FIG. 1B

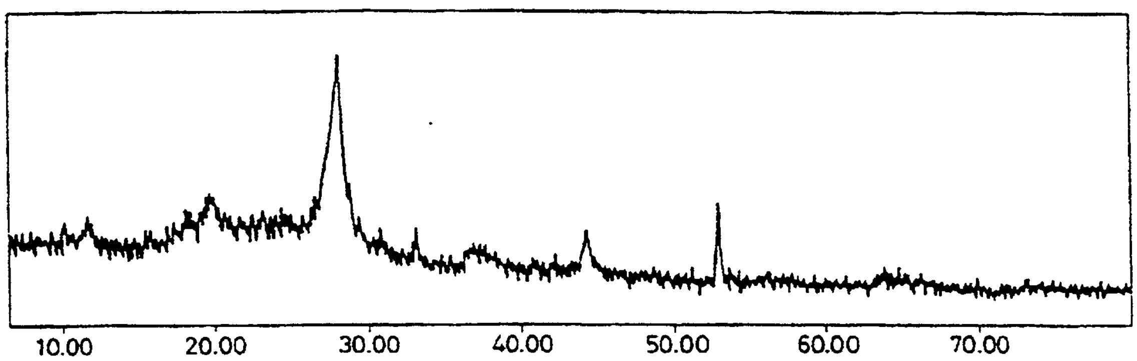
10.00
20.00
30.00
40.00
50.00
60.00
70.00

FIG. 2A

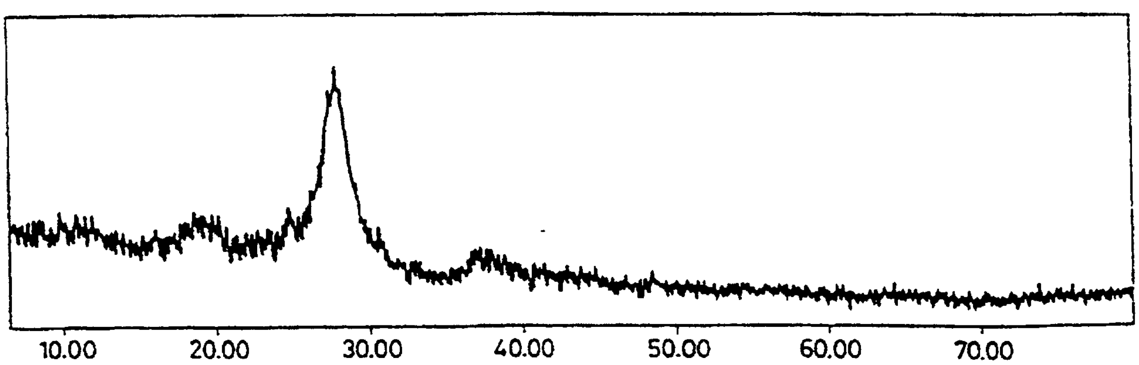
10.00
20.00
30.00
40.00
50.00
60.00
70.00

FIG. 2B

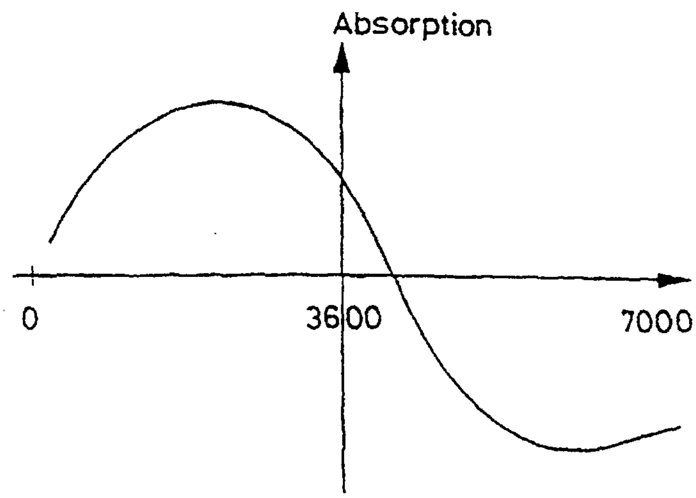


FIG. 3

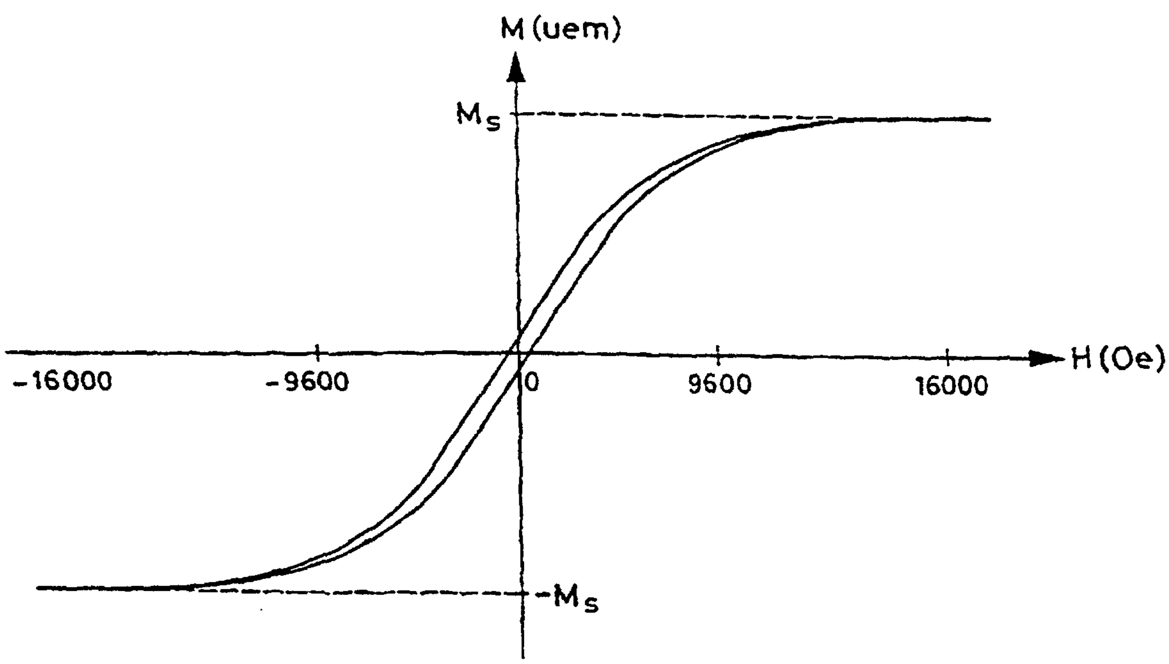


FIG. 4

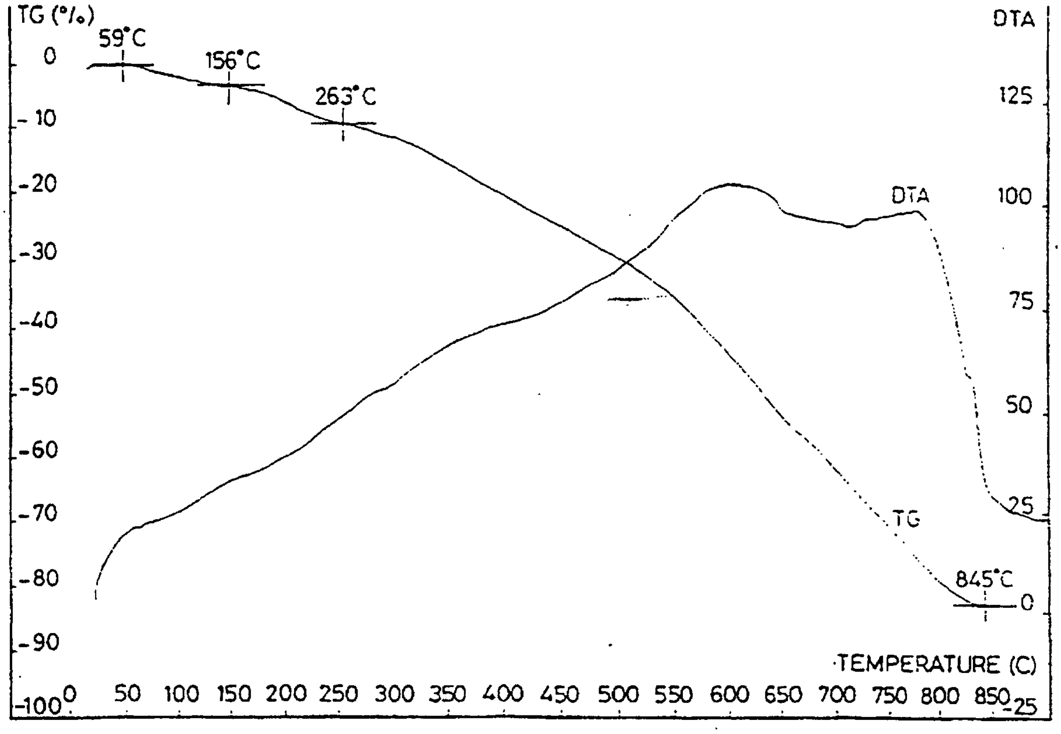
TG (%)
DTA
59°C
156°C
263°C
845°C
0
-10
-20
-30
-40
-50
-60
-70
-80
-90
-100
125
100
75
50
25
0
-25
DTA
TG
TEMPERATURE (C)
0 50 100 150 200 250 300 350 400 450 500 550 600 650 700 750 800 850

FIG. 5A

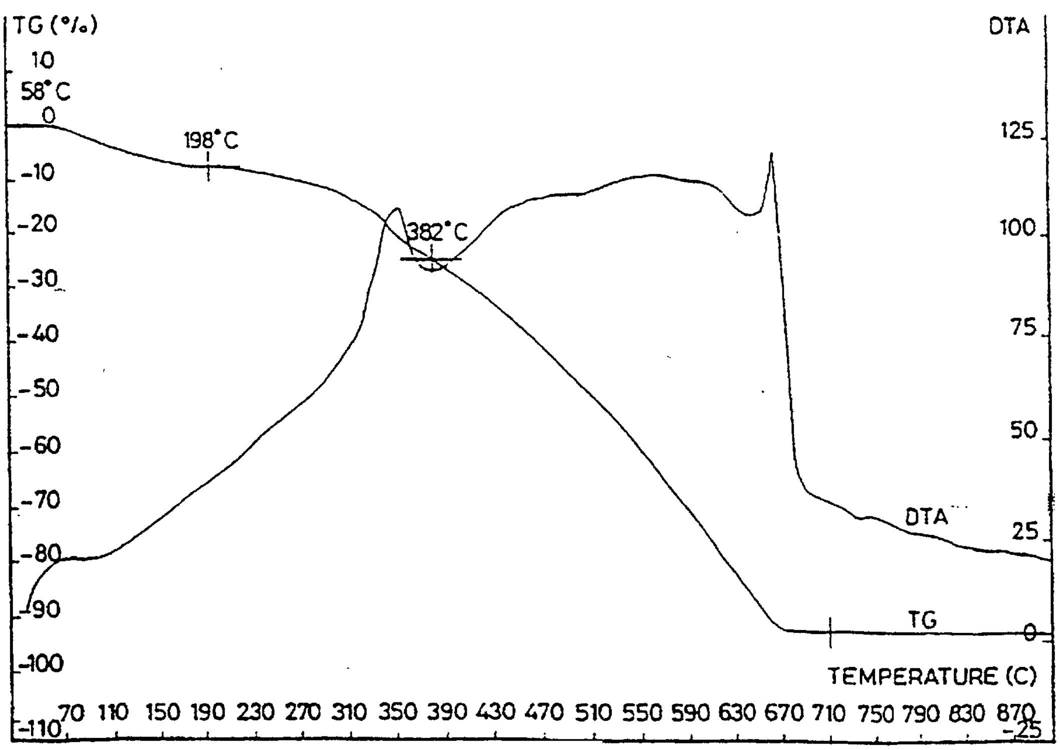
TG (%)
DTA
10
58°C
0
198°C
382°C
-10
-20
-30
-40
-50
-60
-70
-80
-90
-100
-110
125
100
75
50
25
0
-25
DTA
TG
TEMPERATURE (C)
70 110 150 190 230 270 310 350 390 430 470 510 550 590 630 670 710 750 790 830 870

FIG. 5B

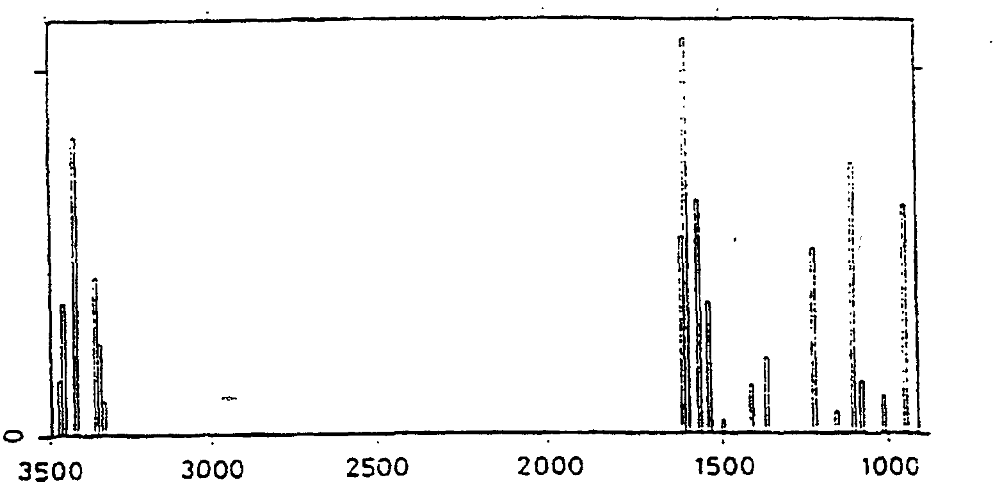


FIG. 6